# EUROPEAN PATENT APPLICATION

(11) **EP 4 179 965 A1**
(43) Date of publication of application: **17.05.2023**
(21) Application number: 22733502.3
(22) Date of filing: 07.05.2022
(51) Int. Cl.: A61B 5/02

(54) **BLOOD PRESSURE MEASUREMENT METHOD AND APPARATUS, AND ELECTRONIC DEVICE AND STORAGE MEDIUM**

(30) Priority: 23.09.2021 CN 202111115404
(71) Applicant: Anhui Huami Health Technology Co., Ltd., Hefei, Anhui 243000 (CN)
(72) Inventor: WANG, Kongqiao, Hefei, Anhui 243000 (CN)
(74) Representative: Canzler & Bergmeier Patentanwälte Partnerschaft mbB
(86) International application number: PCT/CN2022/091502
(87) International publication number: WO 2023/045342

(57) **Abstract**

Provided are a method and apparatus for blood pressure measurement, an electronic device, and a storage medium, which relate to the field of artificial intelligence. The solution comprises: selecting a first photo plethysmo graph (PPG) pulse wave signal from candidate PPG pulse wave signals, wherein the candidate PPG pulse wave signals are PPG pulse wave signals collected from at least two collection channels; in response to the first PPG pulse wave signal not being a PPG pulse wave signal collected from a target collection channel, obtaining a target PPG pulse wave signal for blood pressure measurement by mapping the first PPG pulse wave signal to the PPG pulse wave signal collected from the target collection channel; and obtaining feature information of the target PPG pulse wave signal, and obtaining a blood pressure measurement value based on the feature information.

## Description

### CROSS-REFERENCE TO RELATED APPLICATIONS

This application is based on and claims priority to Chinese Patent Application No. 202111115404.1, filed on September 23, 2021, the content of which is incorporated herein by reference in its entirety.

### TECHNICAL FIELD

The disclosure relate to the field of artificial intelligence, in particular to a method and an apparatus for blood pressure measurement, an electronic device and a storage medium, which may be specifically applied in scenarios on intelligent medical treatment and health detection.

### BACKGROUND

In the related art, the accuracy of pulse wave signals in a photo plethysmo graph (PPG) may change with the tightness of a wearable device and the skin dryness, and there are differences between morphological features and time domain features for PPG pulse wave signals measured at different body parts. Therefore, the field of noninvasive blood pressure measurement still faces great challenges, such as the measurement accuracy.

### SUMMARY

The disclosure provides a method and an apparatus for blood pressure measurement, an electronic device and a storage medium.

According to a first aspect of the disclosure, a method for blood pressure measurement is provided. The method includes: selecting a first photo plethysmo graph (PPG) pulse wave signal from candidate PPG pulse wave signals, in which the candidate PPG pulse wave signals are PPG pulse wave signals collected from at least two collection channels; in response to the first PPG pulse wave signal not being a PPG pulse wave signal collected from a target collection channel, obtaining a target PPG pulse wave signal for blood pressure measurement by mapping the first PPG pulse wave signal to the PPG pulse wave signal collected from the target collection channel; and obtaining feature information of the target PPG pulse wave signal, and obtaining a blood pressure measurement value based on the feature information.

The disclosure collects the candidate PPG pulse wave signals by at least two collection channels, selects the first PPG pulse wave signal from the candidate PPG pulse wave signals, obtains the target PPG pulse wave signal for blood pressure measurement by mapping the first PPG pulse wave signal to the PPG pulse wave signal collected from the target collection channel when the first PPG pulse wave signal is not the PPG pulse wave signal collected from the target collection channel, and obtains the blood pressure measurement value based on the feature information of the target PPG pulse wave signal. In the embodiment of the disclosure, in the process of multi-channel collection, through consistency mapping of the PPG pulse wave signal, the PPG pulse wave signal that is not collected from the target collection channel may be mapped to the required target feature space so that the feature space keeps consistent, which ensures that in a scenario of collecting multi-channel/multi-modal physiological data, even if the selected PPG pulse wave signal is not a signal collected from a specific collection channel, the accurate blood pressure measurement may be achieved, thus improving the applicability and generalization ability of blood pressure measurement. Since the feature space is consistent after mapping, there is no need adjust the blood pressure measurement process or model, thus reducing the measurement complexity and computational memory.

According to another aspect of the disclosure, an apparatus for blood pressure measurement is provided. The apparatus includes: a selection module, configured to select a first photo plethysmo graph (PPG) pulse wave signal from candidate PPG pulse wave signals, in which the candidate PPG pulse wave signals are PPG pulse wave signals collected from at least two collection channels; a mapping module, configured to in response to the first PPG pulse wave signal not being a PPG pulse wave signal collected from a target collection channel, obtain a target PPG pulse wave signal for blood pressure measurement by mapping the first PPG pulse wave signal to the PPG pulse wave signal collected from the target collection channel; and a blood pressure measurement module, configured to obtain feature information of the target PPG pulse wave signal, and obtain a blood pressure measurement value based on the feature information.

According to another aspect of the disclosure, a wearable device is provided, which includes a sensor, a memory and a processor. The sensor is configured to collect a photo plethysmo graph (PPG) pulse wave signal, or synchronously collect a PPG pulse wave signal and an electrocardiogram ECG signal. The processor is configured to read executable program codes stored in the memory and execute a program corresponding to the executable program codes, so as to implement the method for blood pressure measurement according to the first aspect of embodiments of the disclosure.

According to another aspect of the disclosure, an electronic device is provided, which includes a memory and a processor. The processor is configured to read executable program codes stored in the memory and execute a program corresponding to the executable program codes, so as to implement the method for blood pressure measurement according to the first aspect of embodiments of the disclosure.

According to another aspect of the disclosure, a computer-readable storage medium is provided, on which a computer program is stored. When the computer program is executed by a processor, the method for blood pressure measurement according to the first aspect of embodiments of the disclosure is implemented.

According to another aspect of the disclosure, a computer program product is provided, which includes a computer program. When the computer program is executed by a processor, the method for blood pressure measurement according to the first aspect of embodiments of the disclosure is implemented.

According to another aspect of the disclosure, a system for blood pressure measurement is provided, which includes a wearable device and an electronic device wirelessly communicating with the wearable device. The wearable device includes a sensor, and the electronic device includes a memory and a processor. The sensor is configured to collect a first photo plethysmo graph (PPG) pulse wave signal, or synchronously collect a PPG pulse wave signal and an electrocardiogram (ECG) signal. The wearable device is configured to transmit the signal collected by the sensor to the electronic device through wireless communication. The processor on the electronic device is configured to read executable program codes stored in the memory and execute a program corresponding to the executable program codes, so as to implement the method for blood pressure measurement according to the first aspect of embodiments of the disclosure.

It should be understood that, the content described in this section is not intended to identify key or important features of embodiments of the disclosure, nor is it intended to limit the scope of the disclosure. Other features of the disclosure may be readily understood by the following description.

### BRIEF DESCRIPTION OF THE DRAWINGS

The accompanying drawings are used to better understand the present solution, which do not constitute a limitation of the disclosure.
FIG. 1 is a flowchart of a method for blood pressure measurement according to an embodiment of the disclosure.
FIG. 2 is a schematic diagram of feature subspaces of different PPG pulse wave signals.
FIG. 3 is a flowchart of a method for blood pressure measurement according to an embodiment of the disclosure.
FIG. 4 is a flowchart of a method for blood pressure measurement according to an embodiment of the disclosure.
FIG. 5 is a schematic representation of PTT feature extraction.
FIG. 6 is a flowchart of a method for blood pressure measurement according to an embodiment of the disclosure;
FIG. 7 is a flowchart of a method for blood pressure measurement according to an embodiment of the disclosure.
FIG. 8 is a flowchart of a method for blood pressure measurement according to an embodiment of the disclosure.
FIG. 9 is a block diagram of an apparatus for blood pressure measurement according to an embodiment of the disclosure.
FIG. 10 is a block diagram of an electronic device for implementing a method for blood pressure measurement according to an embodiment of the disclosure.

### DETAILED DESCRIPTION

Embodiments of the disclosure may be described in detail, examples of which are illustrated in the drawings. The same or similar reference numerals throughout the specification refer to the same or similar elements or elements having the same or similar functions. Embodiments described herein with reference to drawings are explanatory, serve to explain the disclosure, and are not construed to limit embodiments of the disclosure.

The following describes a method and an apparatus for blood pressure measurement, an electronic device and a storage medium of the disclosure with reference to the accompanying drawings.

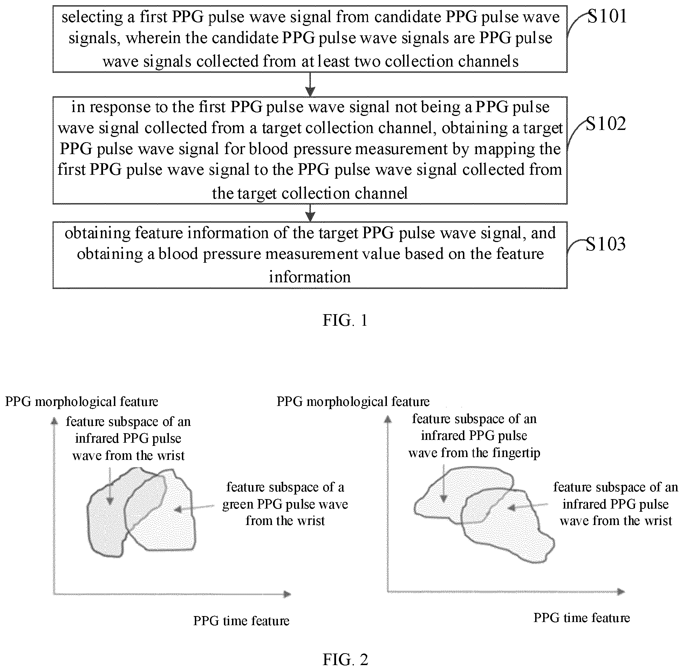

FIG. 1 is a flowchart of a method for blood pressure measurement according to an embodiment of the disclosure. As shown in FIG. 1, the method includes the following steps at S101 to S103.

At S101, a first photo plethysmo graph (PPG) pulse wave signal is selected from candidate PPG pulse wave signals. The candidate PPG pulse wave signals are PPG pulse wave signals collected from at least two collection channels.

In the embodiment of the disclosure, different collection channels may be set at different body parts, and the candidate PPG pulse wave signals may be collected by performing collection of PPG pulse wave signals at different body parts through at least two collection channels. The body parts may include a wrist, a fingertip, an upper arm, and the like. In some embodiments, a green light collection channel may be provided on the wrist, and an infrared light collection channel may be provided on the fingertip.

In practical situations, the signal quality is not good, and the obtained measurement results may not be accurate. Therefore, based on the signal quality of PPG pulse wave signals, a candidate PPG pulse wave signal with good signal quality is selected from the candidate PPG pulse wave signals as the first PPG pulse wave signal.

At S102, in response to the first PPG pulse wave signal not being a PPG pulse wave signal collected from a target collection channel, the first PPG pulse wave signal is mapped to the PPG pulse wave signal collected from the target collection channel, so as to obtain a target PPG pulse wave signal for blood pressure measurement.

The target collection channel may be one of multiple collection channels, for example, the collection channel at the wrist, or the collection channel corresponding to the fingertip. The selection of the target collection channel depends on a type of a blood pressure measurement model. For example, when the blood pressure measurement model is a blood pressure measurement model for a PPG pulse wave signal from the wrist green light, the target collection channel is the collection channel corresponding to the wrist.

Each of the collection channels corresponds to its own collection device, and each collection device has a device identifier. The device identifier may uniquely identify a collection device. In some embodiments, the collected PPG pulse wave signal is carried with a device identifier of a corresponding collection device, so that it may be determined whether the selected PPG pulse wave signal is collected from the target collection channel based on the device identifier carried in the PPG pulse wave signal.

In the related art, it is difficult to simultaneously capture available multi-channel or multi-modal measurement data and make effective use of the data, which is the main reason for the current measurement challenges. This is because morphological features and time domain features of the PPG pulse wave signal are related to a measurement part of the body. The PPG pulse wave signal collected from the fingertip and the pulse wave collected from the wrist have non-overlapping feature subspaces. In practice, there are also difference on features between PPG pulse wave signals of different wavelengths measured at the same part of the body. In a PPG feature space, both the green PPG pulse wave signal and the infrared PPG pulse wave signal that are simultaneously collected from the wrist, also have a feature subspace that is not overlapped with each other, as shown in FIG. 2.

For example, the infrared PPG pulse wave signal collected from the fingertip may be mapped to an infrared PPG pulse wave signal from the wrist by mapping, and the mapped PPG pulse wave signal is consistent with a feature space of a real PPG pulse wave signal collected from the wrist. Alternatively, the infrared PPG pulse wave signal collected from the wrist is mapped to a green PPG pulse wave signal collected from the wrist by mapping.

At 5103, feature information of the target PPG pulse wave signal is obtained, and a blood pressure measurement value is obtained based on the feature information.

The feature information of the target PPG pulse wave signal includes at least one of a pulse transmission time (PTT) feature, a morphological feature and a time domain feature. The PTT feature refers to time when a pulse wave goes from an aortic valve to a certain position of the whole body, and is a feature directly related to a blood pressure. The PTT feature, the morphological feature, and the time domain feature together constitute a feature space of a PPG pulse wave signal. Since these features may reflect characteristics of the target PPG pulse wave signal, the blood pressure measurement value may be predicted based on these feature information. The blood pressure measurement values include a diastolic blood pressure and a systolic blood pressure.

In some embodiments, by extracting feature points of the PPG pulse wave signal, such as a peak value, a trough value, a period and other information, the feature information of the target PPG pulse wave signal may be obtained based on the above feature points, including the PTT feature, the morphological feature, and the time domain feature. In the embodiment of the disclosure, the candidate PPG pulse waves are collected through at least two collection channels, and the first PPG pulse wave signal is selected from the candidate PPG pulse wave signals; in response to the first PPG pulse wave signal not being the PPG pulse wave signal collected from a target collection channel, the first PPG pulse wave signal is mapped to the PPG pulse wave signal collected from the target collection channel to obtain the target PPG pulse wave signal for blood pressure measurement. In the embodiment of the disclosure, in the process of multi-channel collection, through consistency mapping of the PPG pulse wave signal, the PPG pulse wave signal that is not collected from the target collection channel may be mapped to the required target feature space so that the feature space keeps consistent, which ensures that in a scenario of collecting multi-channel/multi-modal physiological data, even if the selected PPG pulse wave signal is not a signal collected from a specific collection channel, the accurate blood pressure measurement may be achieved, thus improving the applicability and generalization ability of blood pressure measurement. Since the feature space is consistent after mapping, there is no need to adjust the blood pressure measurement process or model, thus reducing the measurement complexity and computational memory.

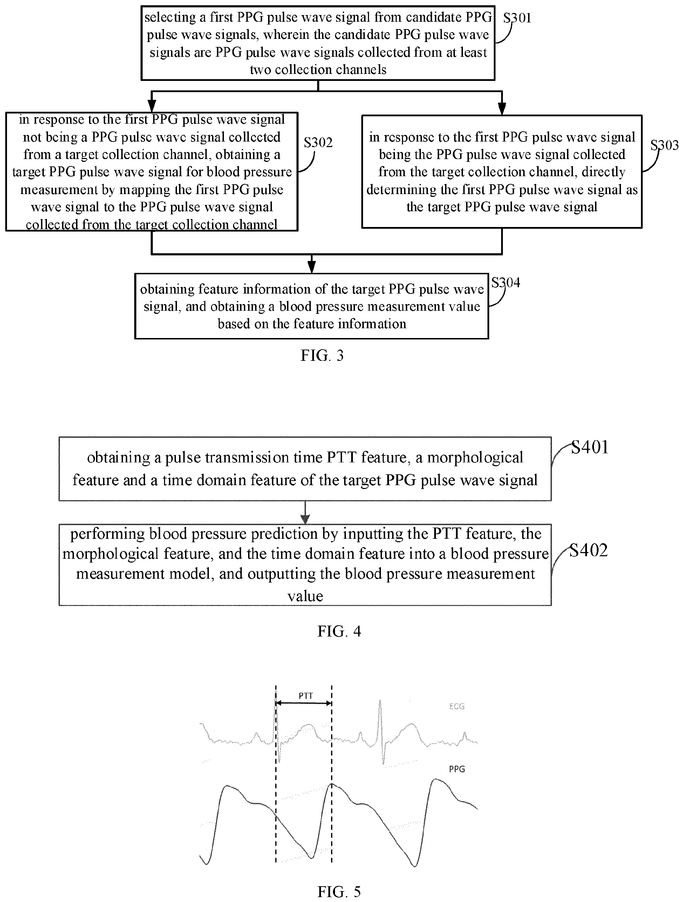

FIG. 3 is a flowchart of a method for blood pressure measurement according to an embodiment of the disclosure. As shown in FIG. 3, the method includes the following steps at S301 to S304.

At S301, a first photo plethysmo graph (PPG) pulse wave signal is selected from candidate PPG pulse wave signals. The candidate PPG pulse wave signals are PPG pulse wave signals collected from at least two collection channels.

For the specific implementation at step S301, reference may be made to relevant introductions in various embodiments of the disclosure, and details are not repeated here.

At S302, in response that the first PPG pulse wave signal is not the PPG pulse wave signal collected from the target collection channel, the first PPG pulse wave signal is mapped to the PPG pulse wave signal collected from the target collection channel, so as to obtain a target PPG pulse wave signal for blood pressure measurement.

A feature space mapping network is pre-trained based on sample pulse waves, and a feature space of the first PPG pulse wave signal is mapped to a feature space of the PPG pulse wave signal collected from the target collection channel through the feature space mapping network. The feature space mapping network is a deep neural network (DNN). In some embodiments, the first PPG pulse wave signal is input into the feature space mapping network, and the feature space mapping is performed on the first PPG pulse wave signal through the feature space mapping network and the target PPG pulse wave signal is output, which may realize there is a consistency with the feature space required by the channel and thus improve the accuracy of blood pressure measurement.

The multiple sample pulse waves are input into the DNN network for training, and the DNN network model may be enabled to find a functional relationship of the mapping network and continuously increase the accuracy of the model judgment by accumulating different data comparisons. Through training, a DNN network that meets conditions may be generated, which may realize the mapping from the non-target feature space to the required feature space. In some implementations, a first DNN1 may be established to map a PPG pulse wave signal of a first color light source collected into a mapped PPG pulse wave signal of a second color light source, the mapped PPG pulse wave signal has uniformity in features with a real PPG pulse wave signal of the second color light source collected at the same part of the body. In another implementations, a second DNN1 may be established to map a PPG pulse wave signal of the second color light source collected to a PPG pulse wave signal of the first color light source, and the mapped PPG pulse wave signal has uniformity in features with a real PPG pulse wave signal of the first color light source collected at the same part of the body. It should be noted that the DNN network may be selected according to actual needs. When the target collection channel corresponds to a PPG pulse wave signal of the second color light source, the first DNN1 is selected; and when the target collection channel corresponds to a PPG pulse wave signal of the first color light source, the second DNN2 is selected.

At S303, in response that the first PPG pulse wave signal is the PPG pulse wave signal collected from the target collection channel, the first PPG pulse wave signal is directly used as the target PPG pulse wave signal.

When the first PPG pulse wave signal responds to the target collection channel, that is, the signal type of the first PPG pulse wave signal is consistent with the signal type corresponding to the blood pressure measurement model, the first PPG pulse wave signal is directly used as the target PPG pulse wave signal.

At S304, feature information of the target PPG pulse wave signal is obtained, and a blood pressure measurement value is obtained based on the feature information.

For the specific implementation at step S304, reference may be made to the relevant introductions in the embodiments of the disclosure, and details are not repeated here.

In the embodiment of the disclosure, in the process of multi-channel collection, when the selected first PPG pulse wave signal is not the PPG pulse wave signal collected from the target collection channel, feature mapping is performed to make the feature space consistent, and blood pressure measurement is performed based on the mapped PPG pulse wave, which ensures that in a scenario of collecting multi-channel/multi-modal physiological data, even if the selected PPG pulse wave signal is not a signal collected from a specific collection channel, the accurate blood pressure measurement may be achieved. When the selected PPG pulse wave signal is the PPG pulse wave signal collected from the target collection channel, the blood pressure measurement is performed directly without mapping, thus improving the timeliness of blood pressure measurement. Through the setup of two branches, the applicability, generalization ability, and flexibility of blood pressure measurement may be improved.

FIG. 4 is a flowchart of a method for blood pressure measurement according to an embodiment of the disclosure. On the basis of the above embodiment, the step of obtaining feature information of the target PPG pulse wave signal, and obtaining a blood pressure measurement value based on the feature information is further explained with reference to FIG. 4. As shown in FIG. 4, the following steps at S401 to S402 are included.

At S401, a pulse transmission time (PTT) feature, a morphological feature and a time domain feature of the target PPG pulse wave signal are obtained.

As an implementation, while the candidate PPG pulse wave signals are collected, an electrocardiogram (ECG) signal is collected synchronously. There is a timing relationship between the ECG/PPG pulse wave signals in the implementation. Therefore, the PTT feature may be obtained based on the synchronously collected ECG signal, the selected first PPG pulse wave signal, and the timing relationship between ECG signals and PPG pulse wave signals synchronously collected. In some embodiments, a moment at one of R-peaks of the ECG signal is first determined as a first occurrence moment, and a moment at a main peak of the first PGG pulse wave signal that first occurs after the first occurrence time is determined as a second occurrence moment. Based on the first and second occurrence time, the PTT feature may be obtained. That is, a time interval between the first occurrence moment and the second occurrence moment is taken as the PTT feature, as shown in FIG. 5.

As another implementation, since a time interval between a main peak and an echo peak of the PPG pulse wave signal has a strong correlation with the PTT, it is also possible to use only the PPG pulse wave signal to perform linear/non-linear fitting on the time interval between the main peak and the echo peak of the PPG pulse wave signal, so that an approximate PTT feature is fitted.

The morphological feature of the PPG pulse wave signal refers to the geometry of a signal. The features points of the PPG pulse wave signal are detected, so as to identify peak (valley) points, starting points and end points of a P-wave, a QRS wave complex, a T-wave of the signal. The morphological feature of the PPG pulse wave signal may be obtained.

The time-domain feature of the PPG pulse wave signal reflect an overall state of the signal, including the following feature parameters: a mean value, a mean square value, an effective value, a peak value, a peak index, a pulse index, a margin index, a skewness index and a kurtosis index of the signal. Through calculation of the above feature parameters, the time domain feature of the PPG pulse wave signal may be obtained.

At S402, the morphological feature, the time domain feature and the PTT feature are input into a blood pressure measurement model to perform blood pressure prediction, and the blood pressure measurement value is output.

In the embodiment of the disclosure, by using the physiological signals obtained by multiple channels, executing a specific strategy for signal consistency calculation, the morphological feature, the time-domain feature and the PTT feature are obtained and are all input into the same blood pressure measurement model for blood pressure prediction. The blood pressure measurement is achieved by the same machine learning/artificial intelligence model.

It should be noted that, the PPG pulse wave signal samples may be collected in advance, and the blood pressure measurement model may be trained based on the training samples. The multiple sample signals are input into the blood pressure measurement model for training. By accumulating different data comparisons, the blood pressure measurement model may be enabled to find a functional relationship from pieces of feature information to blood pressure measurement values and continuously increase the accuracy of the model judgment. Through training, a blood pressure measurement model that meets conditions may be generated, which may convert the obtained morphological feature, the time domain feature and the PTT feature into a blood pressure measurement value.

In the embodiment of the disclosure, the PTT feature, the morphological feature and the time domain feature of the target PPG pulse wave signal are obtained; the morphological feature, the time domain feature and the PTT feature are input into the blood pressure measurement model for blood pressure prediction, and the blood pressure measurement value is output. The embodiment of the disclosure directly obtains the blood pressure measurement value through the blood pressure measurement model by using the PTT feature, morphological feature and time domain feature of the target PPG pulse wave signal. The calculation method of the blood pressure measurement value is simplified, and a more efficient data processing method is provided for the blood pressure measurement.

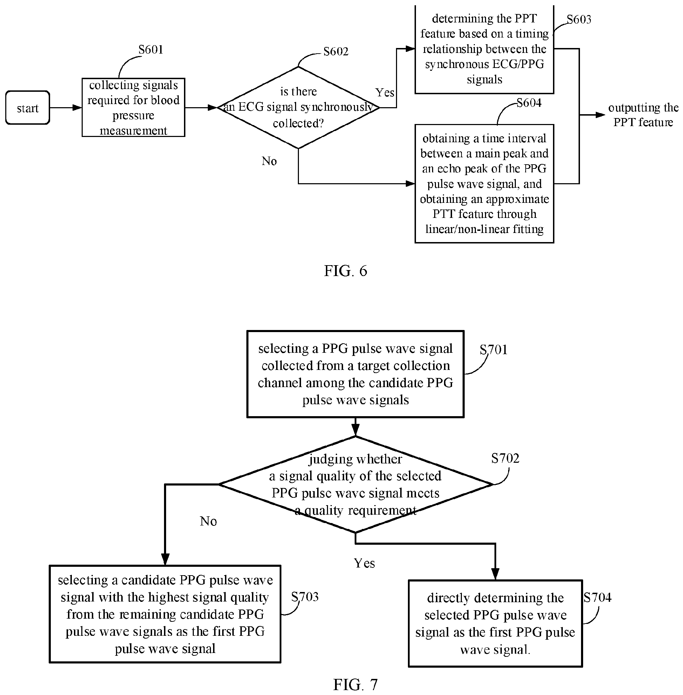

FIG. 6 is a flowchart of a method for blood pressure measurement according to an embodiment of the disclosure. On the basis of the above-mentioned embodiment, the process of collecting the PPT feature of the PPG pulse wave signal is further explained with reference to FIG. 6. As shown in FIG. 6, the following steps at S601 to S604 are included.

At S601, the measurement is started, and physiological signals required for blood pressure measurement are collected.

The required physiological signals, including candidate PPG pulse wave signals and possible ECG signals are collected through at least two collection channels.

S602, it is determined whether to collect the ECG signals synchronously.

When the ECG signals are collected synchronously, it proceeds to S603; and when the ECG signals are not collected synchronously, it proceeds to S604.

Based on a device identifier carried in the signal, it may be determined whether the collected signal is an ECG signal.

Based on the device identifiers of all the collected signals, it may be determined whether to collect the ECG signals synchronously. When the ECG signals are collected synchronously, it proceeds to S603; and when the ECG signals are not collected synchronously, it proceeds to S604.

At S603, the PPT feature is determined based on a timing relationship between the synchronous ECG/PPG signals.

For the specific implementation at step S603, reference may be made to the relevant introductions in the embodiments of the disclosure, and details are not repeated here.

At S604, a time interval between a main peak and an echo peak of the PPG pulse wave signal is obtained, and an approximate PTT feature is obtained through linear/non-linear fitting.

For the specific implementation at step S604, reference may be made to relevant introductions in various embodiments of the disclosure, and details are not repeated here.

In the embodiment of the disclosure, in a scenario of synchronously collecting ECG signals, the PPT feature may be determined according to the timing relationship of the synchronous ECG/PPG signals; in a scenario of not synchronously collecting ECG signals, the approximate PTT feature may be obtained by linear/non-linear fitting. In the embodiments of the disclosure, two strategies for extracting the PTT feature are provided, and the PTT feature extraction may be completed under the condition of whether ECG signals are extracted, which provides a guarantee for the subsequent use of PTT features for predicting blood pressure measurement values.

FIG. 7 is a flowchart of a method for blood pressure measurement according to an embodiment of the disclosure. On the basis of the above-mentioned embodiment, the step of selecting a first PPG pulse wave signal from the candidate PPG pulse wave signals is further explained with reference to FIG. 7. As shown in FIG. 7, the following steps at S701 to S704 are included.

At S701, a PPG pulse wave signal collected from a target collection channel is selected among candidate PPG pulse wave signals.

From the candidate PPG pulse wave signals, the PPG pulse wave signal collected from the target collection channel is determined based on a device identifier of the PPG pulse wave signal.

At S702, it is judged whether a signal quality of the selected PPG pulse wave signal meets a quality requirement.

In some embodiments, the signal quality of the selected PPG pulse wave signal is compared with a standard signal quality. When the signal quality of the selected PPG pulse wave signal is higher than the standard signal quality, it is determined that the signal quality of the selected PPG pulse wave signal meets the quality requirement. When the signal quality of the selected PPG pulse wave signal is lower than or equal to the standard signal quality, it is determined that the signal quality of the selected PPG pulse wave signal does not meet the signaling quality requirement. In some embodiments, a signal strength value representing the signal quality of the selected pulse wave signal may be compared with a standard signal strength value. When the signal strength value is greater than the standard signal strength value, the quality requirement is met. When the signal strength value is less than or equal to the standard signal strength value, the quality requirement is not met.

When the signal quality of the selected PPG pulse wave signal does not meet the quality requirement, it proceeds to step S703. When the signal quality of the selected PPG pulse wave signal meets the quality requirement, it proceeds to step S704.

At S703, a candidate PPG pulse wave signal with the highest signal quality is selected from the remaining candidate PPG pulse wave signals as the first PPG pulse wave signal.

The signal qualities of the remaining candidate PGG pulse wave signals are arranged in descending order, and the PGG pulse wave signal with the highest signal quality is selected as the first PGG pulse wave signal.

At S704, the selected PPG pulse wave signal is directly determined as the first PPG pulse wave signal.

In response to the signal quality of the selected PPG pulse wave signal meeting the quality requirement, the other candidate PPG pulse wave signals do not need to be considered, and the selected PPG pulse wave signal is directly determined as the first PPG pulse wave signal. Since the selected PPG pulse wave signal is collected from the target collection channel, it is not necessary to perform feature mapping on the selected PPG pulse wave signal to improve the timeliness of blood pressure measurement.

In the embodiment of the disclosure, in a scenario of collecting a multi-channel/multi-modal physiological signal, the PPG pulse wave signal for blood pressure measurement is selected based on the signal qualities of the candidate PPG pulse wave signals, so that the PPG pulse wave signal for blood pressure measurement has a good signal quality, which is beneficial to improve the accuracy of blood pressure measurement. In the embodiment of the disclosure, the blood pressure prediction is not performed by directly using the PPG pulse wave signal collected on the target collection channel, which may avoid the problem that when the signal quality of the PPG pulse wave signal is poor, continuing to predict blood pressure results in measurement errors. The PPG pulse wave signal that meets the quality requirement may be selected for blood pressure measurement.

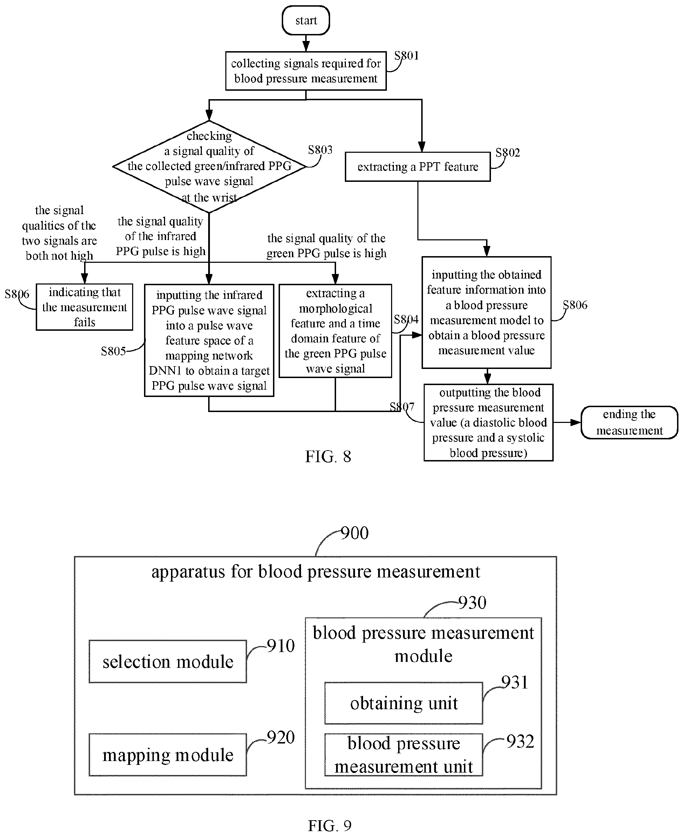

FIG. 8 is a flowchart of a method for blood pressure measurement according to an embodiment of the disclosure. As shown in FIG. 8, based on the method for blood pressure measurement according to the disclosure, the process of blood pressure measurement in an actual application scenario includes the following at steps S801 to S808.

At S801, the measurement is started, and signals required for blood pressure measurement are collected.

In some embodiments, the signal required for blood pressure measurement may include: a PPG pulse wave signal, or a PPG pulse wave signal and an ECG signal. In some embodiments, a green PPG pulse wave signal and an infrared PPG pulse wave signal may be collected separately at the wrist.

At S802, a PTT feature of the PPG pulse wave signal is extracted.

At S803, it is checked a signal quality of the collected PPG pulse wave signals from the wrist green/infrared light.

The collected PPG pulse wave signal from the wrist green light is the PPG pulse wave signal collected by the target collection channel.

When the signal quality of the green PPG pulse wave signal is high, it proceeds to step S804. When the signal quality of the green PPG pulse wave signal is not high, but the signal quality of the infrared PPG pulse wave signal is high, it proceeds to step S805. When the signal qualities of both the green PPG pulse wave signal and the infrared PPG pulse wave signal are not high, it proceeds to step S808.

At S804, a morphological feature and a time domain feature of the green PPG pulse wave signal are extracted.

At S805, the infrared PPG pulse wave signal is input into a pulse wave feature space of a mapping network DNN1 to obtain a target PPG pulse wave signal.

It should be noted that, the target PPG pulse wave signal is a "green light" PPG pulse wave signal mapped that is obtained by mapping the infrared PPG pulse wave signal.

At S806, the obtained feature information is input into a blood pressure measurement model to obtain a blood pressure measurement value.

At S807, the blood pressure measurement value (a diastolic blood pressure and a systolic blood pressure) is output.

At S808, it is indicated that the measurement fails.

In the embodiment of the disclosure, a blood pressure measurement model based on the green PPG pulse wave signal is adopted. The PPG pulse wave signal from the wrist green light and the PPG pulse wave signal from the wrist infrared light are collected as candidate PPG pulse wave signals through two collection channels, and different operation modes are selected according to the quality of the candidate PPG pulse wave signals. When the signal quality of the green PPG pulse wave signal is high, the morphological feature and time domain feature of the green PPG pulse wave signal are extracted. When the signal quality of the green PPG pulse wave signal is not high, but the signal quality of the infrared PPG pulse wave signal is high, the infrared PPG pulse wave signal is input to the pulse wave signal consistency network DNN1 to obtain the "green light" PPG pulse wave signal mapped. When the signal qualities of both the green and infrared PPG pulse wave signals are not high, it may indicate the measurement fails. The obtained morphological features and time domain feature of the PPG pulse wave signal, and the extracted PTT feature are input into the blood pressure measurement model to obtain the blood pressure measurement value. The embodiment of the disclosure collects PPG pulse wave signals of two collection channels, selects PPG pulse wave signals whose quality meets the requirements for signal processing, and calculates the blood pressure measurement value, which improves the accuracy and precision of blood pressure measurement.

FIG. 9 is a structural diagram of an apparatus for blood pressure measurement according to an embodiment of the disclosure. As shown in FIG. 9, the apparatus for blood pressure measurement includes a selection module 910, a mapping module 920 and a blood pressure measurement module 930.

The selection module 910 is configured to select a first photo plethysmo graph (PPG) pulse wave signal from candidate PPG pulse wave signals. The candidate PPG pulse wave signals are PPG pulse wave signals collected from at least two collection channels.

The mapping module 920 is configured to, in response to the first PPG pulse wave signal not being the PPG pulse wave signal collected from the target collection channel, obtain a target PPG pulse wave signal for blood pressure measurement by mapping the first PPG pulse wave signal to the PPG pulse wave signal collected from the target collection channel.

The blood pressure measurement module 930 is configured to obtain feature information of the target PPG pulse wave signal, and obtain a blood pressure measurement value based on the feature information.

In the embodiment of the disclosure, in the process of multi-channel collection, through consistency mapping of the PPG pulse wave signal, the PPG pulse wave signal that is not collected from the target collection channel may be mapped to the required target feature space so that the feature space keeps consistent. This calculation strategy not only effectively utilizes multi-channel/multi-modal physiological data to improve the measurement accuracy, but also adopts only the same measurement model, which reduces the measurement complexity and computational memory, and improves the measurement experience.

It should be noted that, the foregoing explanations in the embodiments of a method for blood pressure measurement are also applicable to this embodiment of the apparatus for blood pressure measurement, which may not be repeated here.

Further, in an implementation of the embodiment of the disclosure, the selection module 710 is further configured to: in response to the first PPG pulse wave signal being the PPG pulse wave signal collected from the target collection channel, directly determine the first PPG pulse wave signal as the target PPG pulse wave signal.

Further, in an implementation of the embodiment of the disclosure, the blood pressure measurement module 930 further includes a collection unit 931 and a blood pressure measurement unit 932.

The collection unit 931 is configured to obtain a pulse transmission time PTT feature, a morphological feature and a time domain feature of the target PPG pulse wave signal.

The blood pressure measurement unit 932 is configured to perform blood pressure prediction by inputting the PTT feature, the morphological feature, and the time domain feature into a blood pressure measurement model, and outputting the blood pressure measurement value.

Further, in an implementation of the embodiment of the disclosure, the first obtaining unit 931 is further configured to: obtain a time interval between a main peak and an echo peak of the target PPG pulse wave signal, and obtaining the PTT feature by fitting the time interval.

Further, in an implementation of the embodiment of the disclosure, the first obtaining unit 931 is further configured to: obtain the PTT feature based on the synchronously collected ECG signal and the candidate PPG pulse wave signals.

Further, in an implementation of the embodiment of the disclosure, the first obtaining unit 931 is further configured to: determine a first occurrence moment for one of R-peaks of the ECG signal; obtain a second occurrence moment for a main peak of the first PPG pulse wave signal that first appears after the first occurrence moment; and the PTT feature based on the first occurrence time and the second occurrence time.

Further, in an implementation of the embodiment of the disclosure, the selection module 910 is further configured to: select one candidate PPG pulse wave signal from the candidate PPG pulse wave signals based on a signal quality of the PPG pulse wave signal as the first PPG pulse wave signal.

Further, in an implementation of the embodiment of the disclosure, the selection module 910 is further configured to: select the PPG pulse wave signal collected from the target collection channel among the candidate PPG pulse wave signals; judge whether the signal quality of the selected PPG pulse wave signal meets a quality requirement; in response that the signal quality of the selected PPG pulse wave signal does not meet the quality requirement, select a candidate PPG pulse wave signal with the highest signal quality from the remaining candidate PPG pulse wave signals as the first PPG pulse wave signal; and in response that the signal quality of the selected PPG pulse wave signal meets the quality requirement, directly determine the selected PPG pulse wave signal as the first PPG pulse wave signal.

According to embodiments of the disclosure, the disclosure also provides an electronic device, a readable storage medium, and a computer program product.

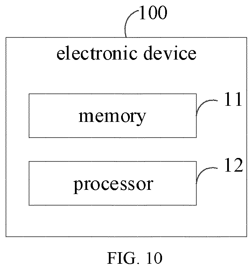

FIG. 10 shows a schematic block diagram of an example electronic device 100 that may be used to implement embodiments of the disclosure. The electronic device is intended to represent various forms of digital computers, such as laptops, desktops, workstations, personal digital assistants, servers, blade servers, mainframe computers, and other suitable computers. The electronic device may also represent various forms of mobile devices, such as personal digital processors, cellular phones, smart phones, wearable devices, and other similar computing devices. A part of the functions of the above electronic device may be implemented on wearable devices, and another part of the functions may be implemented on other mobile devices. The other mobile devices may be, for example, mobile terminals such as cellular phones and smart phones. The wireless communication may be performed between the wearable devices and the other mobile devices, such as short-range wireless communication. The components shown herein, their connections and relationships, and their functions are only as an example, and are not intended to limit the implementations of the disclosure described and/or claimed herein.

As shown in FIG. 10, the electronic device includes a memory 11, a processor 12, and a computer program stored in the memory 11 and running on the processor 12. When the processor 12 executes the program, the aforementioned method for blood pressure measurement is implemented.

In addition, the terms "first" and "second" are only used for purposes of description, and are not intended to indicate or imply relative importance or the number of indicated technical features. Thus, the feature defined with "first", "second" may expressly or implicitly include at least one of that feature. In the description of the disclosure, "plurality" means at least two, such as two, three, etc., unless expressly and specifically defined otherwise.

In the description of this specification, description with reference to the terms "an embodiment," "some embodiments," "an example," "a specific example," or "some examples", etc., mean a particular feature, structure, material, or characteristic described in connection with the embodiment or example is included in at least one embodiment or example of the disclosure. In this specification, schematic representations of the above terms are not necessarily directed to the same embodiment or example. Furthermore, the particular features, structures, materials or characteristics described may be combined in any suitable manner in any one or more embodiments or examples. In addition, different embodiments or examples and features of different embodiments or examples described in the specification may be combined by those skilled in the art without mutual contradiction.

Although the embodiments of the disclosure have been shown and described above, it should be understood that the above-mentioned embodiments are exemplary and should not be construed as limiting the disclosure. Embodiments are subject to variations, modifications, substitutions and variations.

## Claims

1. A method for blood pressure measurement, comprising:
selecting a first photo plethysmo graph PPG pulse wave signal from candidate PPG pulse wave signals, wherein the candidate PPG pulse wave signals are PPG pulse wave signals collected from at least two collection channels;
in response to the first PPG pulse wave signal not being a PPG pulse wave signal collected from a target collection channel, obtaining a target PPG pulse wave signal for blood pressure measurement by mapping the first PPG pulse wave signal to the PPG pulse wave signal collected from the target collection channel; and
obtaining feature information of the target PPG pulse wave signal, and obtaining a blood pressure measurement value based on the feature information.

2. The method of claim 1, further comprising:
in response to the first PPG pulse wave signal being the PPG pulse wave signal collected from the target collection channel, directly determining the first PPG pulse wave signal as the target PPG pulse wave signal.

3. The method of claim 1 or 2, wherein obtaining feature information of the target PPG pulse wave signal, and obtaining the blood pressure measurement value based on the feature information comprises:
obtaining a pulse transmission time PTT feature, a morphological feature and a time domain feature of the target PPG pulse wave signal; and
performing blood pressure prediction by inputting the PTT feature, the morphological feature, and the time domain feature into a blood pressure measurement model, and outputting the blood pressure measurement value.

4. The method of claim 3, wherein obtaining the PTT feature of the target PPG pulse wave signal comprises:
obtaining a time interval between a main peak and an echo peak of the target PPG pulse wave signal, and obtaining the PTT feature by fitting the time interval.

5. The method of claim 3, wherein obtaining the PTT feature of the target PPG pulse wave signal comprises:
obtaining the PTT feature based on an electrocardiogram ECG signal synchronously collected and the target PPG pulse wave signal.

6. The method of claim 5, wherein obtaining the PTT feature based on the electrocardiogram ECG signal synchronously collected and the target PPG pulse wave signal comprises:
determining a first occurrence moment for one of R-peaks of the ECG signal;
obtaining a second occurrence moment for a main peak of the first PPG pulse wave signal that first appears after the first occurrence moment; and
obtaining the PTT feature based on the first occurrence time and the second occurrence time.

7. The method of claim 1 or 2, wherein selecting the first PPG pulse wave signal from the candidate PPG pulse wave signals comprises:
selecting one candidate PPG pulse wave signal from the candidate PPG pulse wave signals based on a signal quality of the PPG pulse wave signal as the first PPG pulse wave signal.

8. The method of claim 7, wherein selecting one candidate PPG pulse wave signal from the candidate PPG pulse wave signals based on the signal quality of the PPG pulse wave signal as the first PPG pulse wave signal comprises:
selecting the PPG pulse wave signal collected from the target collection channel among the candidate PPG pulse wave signals;
judging whether the signal quality of the selected PPG pulse wave signal meets a quality requirement;
in response that the signal quality of the selected PPG pulse wave signal does not meet the quality requirement, selecting a candidate PPG pulse wave signal with the highest signal quality from the remaining candidate PPG pulse wave signals as the first PPG pulse wave signal; and
in response that the signal quality of the selected PPG pulse wave signal meets the quality requirement, directly determining the selected PPG pulse wave signal as the first PPG pulse wave signal.

9. The method of claim 1 or 2, wherein obtaining the target PPG pulse wave signal for blood pressure measurement by mapping the first PPG pulse wave signal to the PPG pulse wave signal collected from the target collection channel comprises:
inputting the first PPG pulse wave signal into a feature space mapping network, wherein the feature space mapping network is configured to map a feature space of the first PPG pulse wave signal to a feature space of the PPG pulse wave signal collected from the target collection channel; and
outputting the target PPG pulse wave signal by performing feature space mapping on the first PPG pulse wave signal through the feature space mapping network.

10. An apparatus for blood pressure measurement, comprising:
a selection module, configured to select a first photo plethysmo graph PPG pulse wave signal from candidate PPG pulse wave signals, wherein the candidate PPG pulse wave signals are PPG pulse wave signals collected from at least two collection channels;
a mapping module, configured to in response to the first PPG pulse wave signal not being a PPG pulse wave signal collected from a target collection channel, obtain a target PPG pulse wave signal for blood pressure measurement by mapping the first PPG pulse wave signal to the PPG pulse wave signal collected from the target collection channel; and
a blood pressure measurement module, configured to obtain feature information of the target PPG pulse wave signal, and obtain a blood pressure measurement value based on the feature information.

11. A wearable device, comprising a sensor, a memory and a processor, wherein
the sensor is configured to collect a photo plethysmo graph PPG pulse wave signal, or synchronously collect a PPG pulse wave signal and an electrocardiogram ECG signal;
the processor is configured to read executable program codes stored in the memory and execute a program corresponding to the executable program codes, so as to implement the method according to any one of claims 1 to 9.

12. The wearable device of claim 11, wherein the sensor is a PPG sensor or a multimodal sensor with at least two channels.

13. An electronic device, comprising a memory and a processor;
wherein the processor is configured to read executable program codes stored in the memory and execute a program corresponding to the executable program codes, so as to implement the method according to any one of claims 1 to 9.

14. The electronic device of claim 13, wherein the electronic device is a mobile terminal or a remote server.

15. A computer-readable storage medium on which a computer program is stored, wherein when the computer program is executed by a processor, the method according to any one of claims 1 to 9 is implemented.

16. A computer program product comprising a computer program, wherein when the computer program is executed by a processor, the method according to any one of claims 1 to 9 is implemented.

17. A system for blood pressure measurement, comprising a wearable device and an electronic device wirelessly communicating with the wearable device; wherein the wearable device comprises a sensor, and the electronic device comprises a memory and a processor;
the sensor is configured to collect a first photo plethysmo graph PPG pulse wave signal, or synchronously collect a PPG pulse wave signal and an electrocardiogram ECG signal;
the wearable device is configured to transmit the signal collected by the sensor to the electronic device through wireless communication;
the processor on the electronic device is configured to read executable program codes stored in the memory and execute a program corresponding to the executable program codes, so as to implement the method according to any one of claims 1 to 9.

18. The system of claim 17, wherein the sensor is a PPG sensor or a multimodal sensor with at least two channels.

19. The system of claim 17, wherein the electronic device is a mobile terminal or a remote server.
